(19) 

**Europäisches Patentamt**
**European Patent Office**
**Office européen des brevets**

(11) **EP 2 028 621 A1**

(12) **EUROPEAN PATENT APPLICATION**

(43) Date of publication:
**25.02.2009 Bulletin 2009/09**

(21) Application number: **07114581.7**

(22) Date of filing: **20.08.2007**

(51) Int Cl.:
*G06T 7/60* (2006.01)   *A61B 6/00* (2006.01)

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(71) Applicant: **Agfa HealthCare NV**
**2640 Mortsel (BE)**

(72) Inventors:
 • **Cresens, Marc**
  **2640 Mortsel (BE)**
 • **Wellens, Geert**
  **2640 Mortsel (BE)**
 • **Bertens, Tom**
  **2640 Mortsel (BE)**

(54) **System and method for information embedding and extraction in phantom targets for digital radiography systems**

(57)    A system and method for embedding in a phantom target used in digital radiography systems information specific to the phantom target, such as serial number, phantom target type, precision landmarks, composition, physical properties of the dedicated targets and the like. The information is encoded and represented by small holes in the phantom target that can easily and unambiguously be detected by decoding-software acting on the radiation image of the phantom target. A look-up table or any other flexible data structure defines the functionality of the holes detected based on their image-location. Regions of interest can be located based on an internal coordinate system defined accurately by the sub-pixel position of the hole centres.

Fig. 2

EP 2 028 621 A1

**Description**

FIELD OF THE INVENTION

The present invention relates to acceptance testing and periodic image quality testing of digital computed (CR) or direct (DR) radiography systems (for a comparison between both, see Robert Bruce, "CR versus DR - what are the options?", www.auntminnie.com). Both systems will be referred to in the following as digital radiography systems.

More specifically the invention relates to the identification of the phantom target and its characteristics used during quality control of the system. It is of the utmost importance that no errors in this identification nor in the accurate location of the holes are made, since this inevitably affects the relevance and the accuracy of the image quality results analysed.

A phantom target is a dummy target used in an image capturing system to test the behaviour of the system. The phantom target has known characteristics, and it contains clearly detectable sub-targets that each form a region of interest (ROI), and each with characteristics such that the image quality performance can be analysed in all aspects from the phantom target image. See EP 1,369,084 and EP 1,380,259 for examples of phantom targets.

BACKGROUND OF THE INVENTION

Image quality performance assessment (QA) and performance control (QC) for digital radiography systems (computed radiography CR or direct radiography DR) are of crucial importance within the context of medical diagnostic imaging. QA/QC testing and reporting of the results for digital X-ray projection image acquisition systems has globally evolved from a moral obligation status towards mandatory requirements, imposed by local health care regulations over the last decade.

Quality control can be performed at several instances during the life-cycle of a digital radiography system. Manufacturers of digital computed radiography equipment can integrate image quality performance testing as part of their final QC-testing procedures, performed prior to customer shipment. Also, hospitals can perform acceptance testing. This acceptance testing relies on the results from image quality performance testing, executed after initial delivery, move, reconfiguration or repair of the image acquisition system or its vital components. Furthermore, periodic quality control testing, also referred to as constancy testing, can be part of a quality assurance program which tracks the image quality performance of computed radiography systems by reporting their time-consecutive QC-results, collected on a regular basis (daily, weekly, monthly, ...) to survey the system performance status relative to the image quality requirements and also to gather input for preventive maintenance scheduling.

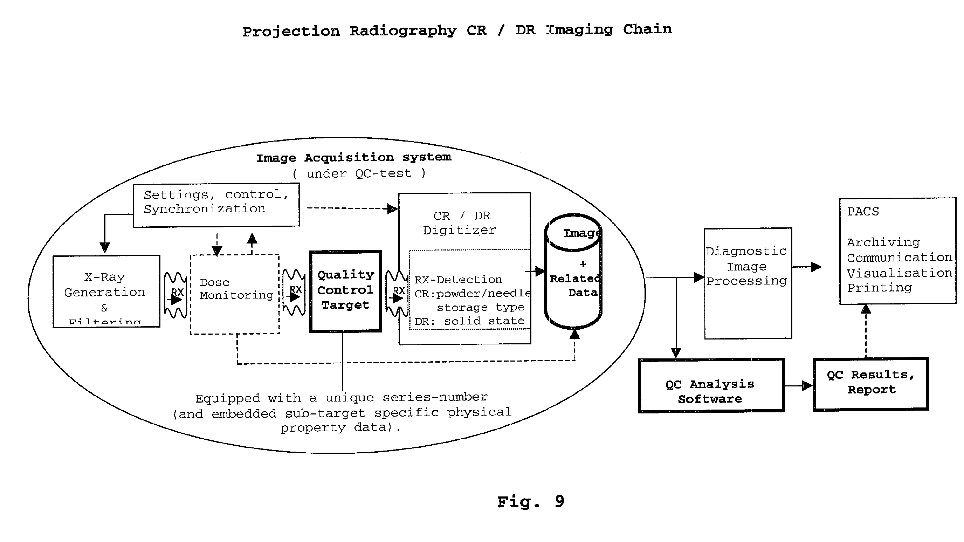

As shown in fig. 9 an image acquisition system for computed radiography is composed of various, linked subcomponents e.g. a console, a generator, an X-ray source, a dose monitor (optional) and a detector/digitizer.

The X-ray source is driven by the generator, receiving commands, settings and synchronization from the console. The generator settings, the tube assembly and the external filters, positioned in the beam-path near the X-ray tube, determine the energy spectrum of the generated photons used for projection imaging. An optional dose monitor inside the beam-path can provide accurate exposure information. An absorption shadow of an object (quality control phantom target, patient), present in the optical path during exposure, is projected onto a X-ray sensitive detection surface, external to (storage phosphor medium based for CR) or integrated inside (solid state sensor based for DR) a digitizer.

The detectors used in the digital radiography system may be powder phosphor plates or needle image plates (needle IP), direct radiography detectors (amorphous silicon, amorphous selenium, Cmos, phosphor detector arranged for direct radiography etc.) or the like. A phosphor plate or needle image plate is commonly conveyed in a cassette and is not part of the read out system.

The digitizer converts the object's impinging X-ray shadow, captured and stored by the detector, into a digital image. Additional information, related to the image captured, such as: time, location, system configuration, system settings, imaging mode, exposure conditions, spectrum, dose, ..., which can be relevant for routing, processing and storage of the generated image can be attached to the image data file. The obtained raw images, if used for medical purposes, are subject to dedicated diagnostic image processing to make them optimally suited for soft- or hardcopy inspection by radiologists or for computer aided detection purposes. The processed images can be visualized, archived, communicated, printed etc. on e.g. a Picture Archiving and Communication System (PACS). An embodiment of a digitizer is described in US 6,369,402.

The scanning technique in the digitizer could be flying-spot or one line at a time. See e.g. R. Schaetzing, R. Fasbender, P. Kersten, "New high-speed scanning technique for Computed Radiography", Proc. SPIE 4682, pp. 511-520.

The image quality performance testing of the image acquisition system, the front-end of the projection radiography imaging chain, performed during acceptance testing or constancy testing does not require X-ray exposure of human or animal beings.

Image quality performance testing involves acquisition and processing of digital images according to predetermined, well defined procedures and X-ray exposure conditions (sequence, timing, geometry, spectrum, dose, ...) by projection imaging one or multiple, dedicated quality control targets, also referred to as phantom targets, positioned in the beam-path between the X-ray source and the detection surface. These QC-targets can be composed of various objects and materials, pattern-wise arranged and spatially distributed inside the target such that the target is optimally suited as a test-object to produce images under exposure conditions, representative for the medical use of the equipment.

The obtained image data and the related information, contained inside the QC-target image, can be processed by dedicated QC-analysis software according to specific algorithms. These algorithms are designed to discriminate and measure the various, characteristic image quality performance parameters, representing the imaging capabilities of the system under test, and relate the calculated performance status to the required image quality criteria, proposed or mandatory for medical use. The QC-test results and comparative findings can be automatically reported and these reports can be archived in a Picture Archiving and Communication System (PACS) or in a dedicated QC-document data base (repository).

Since image acquisition systems for computed radiography are composed of various linked sub-components, the end-resulting image quality performance of the overall system will be determined by the individual image quality performance contributions of the various sub-components, part of the projection imaging chain. Image sharpness for instance, a typical important image quality performance parameter often analyzed, not only depends on the digitizer's modulation transfer function but is also influenced by the selected X-ray tube focus-size and by spatial blurring in the detector-plane. This spatial blurring can occur due to X-ray scatter inside the detector as a function of detector composition and photon spectrum or by strayed stimulation-light during plate-readout (CR).

For this reason overall image quality performance testing often breaks up into multiple, separate QC-tests to evaluate the proper operation of the various system components, each executed under well controlled geometry and exposure conditions according to predetermined and well defined test procedures.

Since the QC-target, a prerequisite to create QC-target images, is an integral part of the image acquisition system during QC-testing, it will, like the other system-components that are part of the imaging chain, have an impact on the properties of the projected target-shadow, of which the QC-target image is generated and of which the image quality performance parameters are derived by calculations.

Image quality performance acceptance criteria are established by QC-analysis of QC-target images, captured from a nominal reference QC-target for each typical, representative system configuration under well controlled exposure conditions. During these tests to establish the reference acceptance criteria for a given image acquisition system only system components showing nominal performance should be part of the imaging chain. These image quality performance acceptance criteria found can be used to evaluate the performance status of a medical diagnostic image acquisition system at the end of the manufacturing chain and out in the field.

The first step in testing is assuring that the input really is what it is supposed to be. Existing systems use bar code labels, or other inserts or add-ons physically attached to the phantom target. The information contained in these artifacts has to be extracted by means of other decoding means and processes. The X-ray image of the phantom target then contains no physically embedded information on the phantom target, and errors cannot be completely excluded.

- By accident a wrong phantom target can be used.
- The phantom target could be wrongly positioned.
- Scanning speed distortions in the fast and in the slow scan directions could lead to not finding or mis-locating the regions of interest (ROIs) and the precision landmarks.

It is an object of the present invention to provide a method for unambiguously identifying the phantom target, the location of its sub-targets and their associated physical properties as well as a method to extract all that embedded information from the phantom target's digital X-ray image.

SUMMARY OF THE INVENTION

The above-mentioned effects are realised by a method and system for coding information having the specific features set out in claim 1 and claim 12, and a method and system for extracting information having the specific features set out in claim 8 and 19. Specific features for preferred embodiments of the invention are set out in the dependent claims.

A system and method are provided for embedding information in a phantom target used in quality control of digital radiography systems, the method characterized in that the information contained in said phantom target is coded; said code is represented by detectable alterations in the thickness of the absorbing layer of said phantom target; and a look-up table or other flexible data structure governs the functional meaning associated with the presence, absence and image-location of said alterations.

Another system and method are provided for extracting physically embedded, encoded information from a phantom target used in digital radiography systems, the method characterized in that alterations in the thickness of the absorbing layer of said phantom target are detected by analyzing the digital X-ray image of said phantom target; the geometrical gravity centre of said detected alterations is localized by means of sub-pixel geometrical gravity centre determination; and said geometrical gravity centres are checked with a look-up table or other flexible data structure governing the functional meaning associated with the presence, absence and image-location of said alterations;

BRIEF DESCRIPTION OF THE DRAWINGS

- Fig. 1 gives a flow chart of the hole detection and the sub-pixel geometrical gravity centre calculation.
- Fig. 2 gives an example of the encoded information structures in a phantom target.
- Fig. 3 illustrates the kernel and masks used in the isotropic gradient calculation.
- Fig. 4 illustrates the isotropic gradient image analysis.
- Fig. 5 illustrates the background mask and hole mask used in the geometrical gravity centre calculation.
- Fig. 6 illustrates the initial geometrical gravity centre ROI.

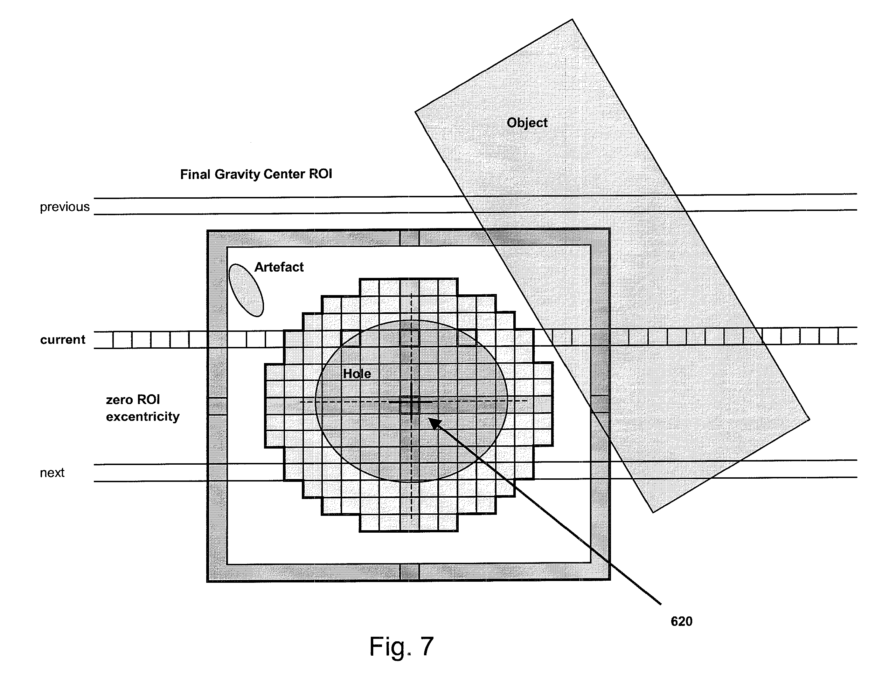

- Fig. 7 illustrates the final geometrical gravity centre ROI.

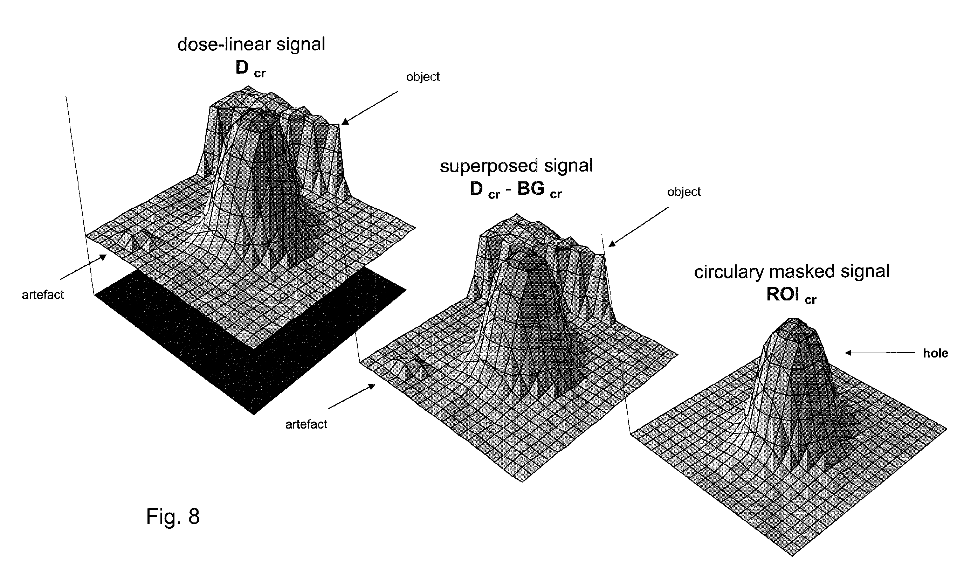

- Fig. 8 gives a visualisation of ROI signal preprocessing prior to geometrical gravity centre calculation.
- Fig. 9 shows an image acquisition system for computed radiography.

DETAILED DESCRIPTION OF THE INVENTION

The current invention addresses above mentioned problems by embedding the necessary information in the phantom target itself, so that it can be automatically recognized and decoded by the analysis software.

Embedding information in the phantom target could be achieved in many ways. It would be possible for instance to change the composition of the material of the phantom target in a controlled and meaningful way, and extract that information from the X-ray image.

The preferred embodiment uses holes drilled in an X-ray absorber layer to encode the information, resulting in a cluster of higher image-signals, i.e. there is less absorption of the incoming X-rays at the location of the holes which is a clear indication that the supposed artifact is artificial. This prevents the detection software from analyzing stains or dust particles on the phantom target since these always will give lower image-signals due to their locally elevated X-ray absorption. In the preferred embodiment said holes are circular and standardized to a predetermined diameter to ease detection. The information is coded in the relative location of the holes, their presence or absence and their sub-pixel geometrical gravity centre image-location (the geometrical gravity centre of an object is the point around which the volume of the object is evenly distributed). These holes must be sufficiently small not to disturb the X-ray image. Circular holes can easily and accurately be drilled at low cost and with a high degree of reproducibility into the phantom target's X-ray absorbing layer by means of Computer Numerical Control (CNC) techniques. Circular holes also cause isotropic image disturbances which again facilitates their detection.

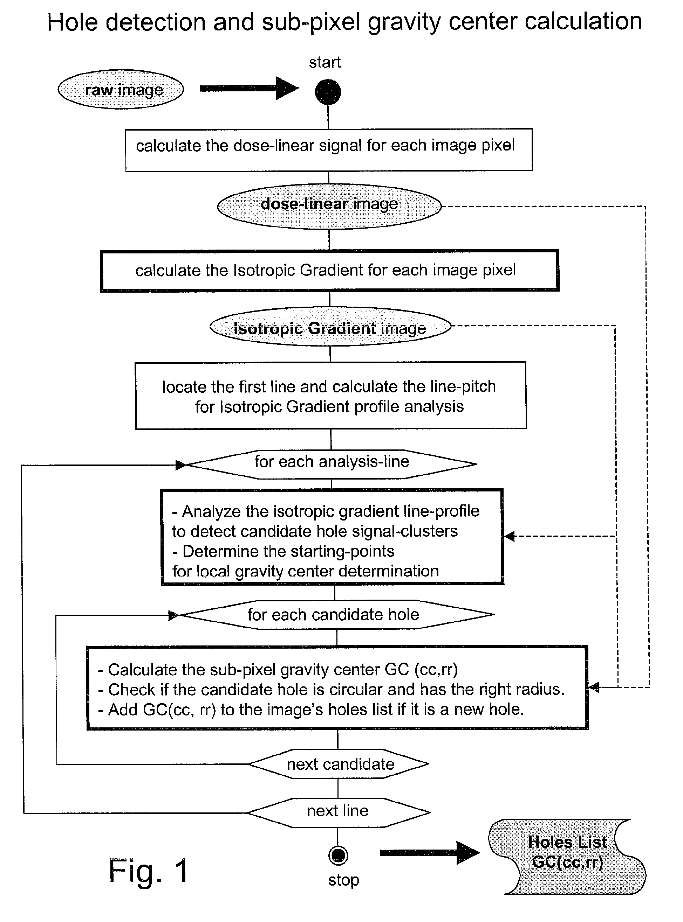

The flow chart for hole detection and sub-pixel geometrical gravity centre calculation is given in Fig. 1, and is described now in more detail.

The first step in interpretation is to scan the digital X-ray image and locate all the holes in the image. First the phantom image is dose-linearised, i.e. the data are decompressed by converting square root or logarithmic to linear, and the zero-dose offset is subtracted. Then the isotropic gradient image is calculated, on which edge analysis can be performed. Knowing that a hole gives a small high contrast spot of low absorption signals on the image, the gradient image will show a small but clear circle indicating the edge of the hole.

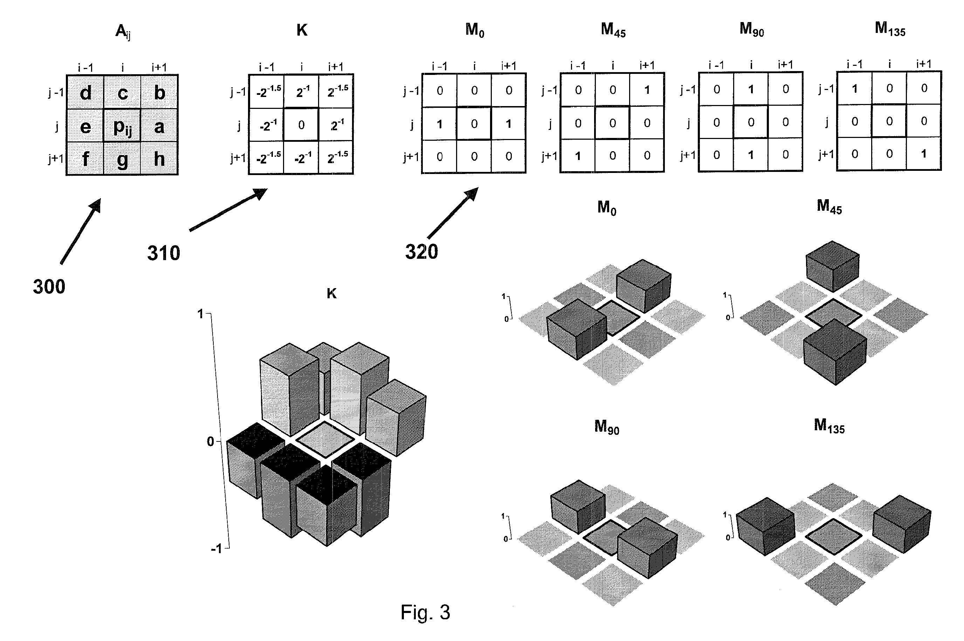

The calculation of said isotropic gradient image is illustrated in Fig. 3. For every pixel in the image a local area **300** is defined as a square containing 9 pixels, the original pixel being its centre. The Local Area Image Data ($A_{ij}$) of pixel $p_{ij}$ at row i and column j is a 3x3 matrix containing the value of the pixel and the values of all its neighbours, where the variables a to h represent the pixel values of the local area:

$$A_{ij} = \begin{pmatrix} d & c & b \\ e & p_{ij} & a \\ f & g & h \end{pmatrix}$$

A Spatial Convolution Kernel (K) **310** is defined as a 3x3 matrix and contains the relative weights of every neighbouring pixel. A possible embodiment looks like this:

$$K = \begin{pmatrix} K_d & K_c & K_b \\ K_e & 0 & K_a \\ K_f & K_g & K_h \end{pmatrix} = \begin{pmatrix} -2^{-1.5} & 2^{-1} & 2^{-1.5} \\ -2^{-1} & 0 & 2^{-1} \\ -2^{-1.5} & -2^{-1} & 2^{-1.5} \end{pmatrix}$$

The values $K_a$ to $K_h$ are chosen such that the difference in distance from the centre is compensated. Thus the values $K_d$ and $K_f$ are a factor $\sqrt{2}$ smaller than the values $K_e$ and $K_g$, and the values $K_b$ and $K_h$ are a factor $\sqrt{2}$ smaller than the values $K_c$ and $K_a$.

Four Spatial Convolution Masks ($M_0$, $M_{45}$, $M_{90}$, $M_{135}$) **320** are defined as 3x3 matrices. The subscript indicates the angle of the measuring direction of the mask with the horizontal axis.

$$M_{0°} = \begin{pmatrix} 0 & 0 & 0 \\ 1 & 0 & 1 \\ 0 & 0 & 0 \end{pmatrix} \qquad M_{45°} = \begin{pmatrix} 0 & 0 & 1 \\ 0 & 0 & 0 \\ 1 & 0 & 0 \end{pmatrix} \qquad M_{90°} = \begin{pmatrix} 0 & 1 & 0 \\ 0 & 0 & 0 \\ 0 & 1 & 0 \end{pmatrix} \qquad M_{135°} = \begin{pmatrix} 1 & 0 & 0 \\ 0 & 0 & 0 \\ 0 & 0 & 1 \end{pmatrix}$$

The isotropic gradient $IG_{ij}$ in pixel $p_{ij}$ is now calculated as:

$$IG_{ij} = \frac{\sqrt{\left(A_{ij} \otimes (K \times M_0)\right)^2 + \left(A_{ij} \otimes (K \times M_{90})\right)^2} + \sqrt{\left(A_{ij} \otimes (K \times M_{45})\right)^2 + \left(A_{ij} \otimes (K \times M_{135})\right)^2}}{2}$$

The symbol $\otimes$ represents matrix convolution, and the symbol $\times$ represents the element product of matrix elements.

In this particular embodiment this formula translates to:

$$IG_{ij} = \frac{\sqrt{(a-e)^2 + (c-g)^2} + \sqrt{\dfrac{(b-f)^2 + (h-d)^2}{2}}}{4}$$

This isotropic gradient implementation gives a vivid spatial response due to the small kernel size. There is a zero phase shift with regard to the input image due to the centre-symmetrical differentials, and also a good noise filtering due to the use of all the neighbouring pixels, i.e. including the corner pixels of the local area.

This way the isotropic gradient of every pixel in the image is calculated, resulting in an isotropic gradient image. This isotropic gradient image now will be further analysed to determine the centre-position of every hole in the image. Thereto every $n^{th}$ row (or column) of the isotropic gradient image is scanned by the software, where $n$ is chosen such that it is guaranteed that every hole is at least encountered once. This is achieved when the physical distance covered by $n$ rows (or columns) is smaller than the predetermined diameter of the holes. In this particular embodiment this diameter is 1 mm, and the physical distance covered by $n$ rows (or columns) is 0.7 mm. If a hole is encountered a second time, during the edge profile analysis of the next analysis line, it will be regarded as the same hole due to the coincidence of both calculated geometrical gravity centre locations.

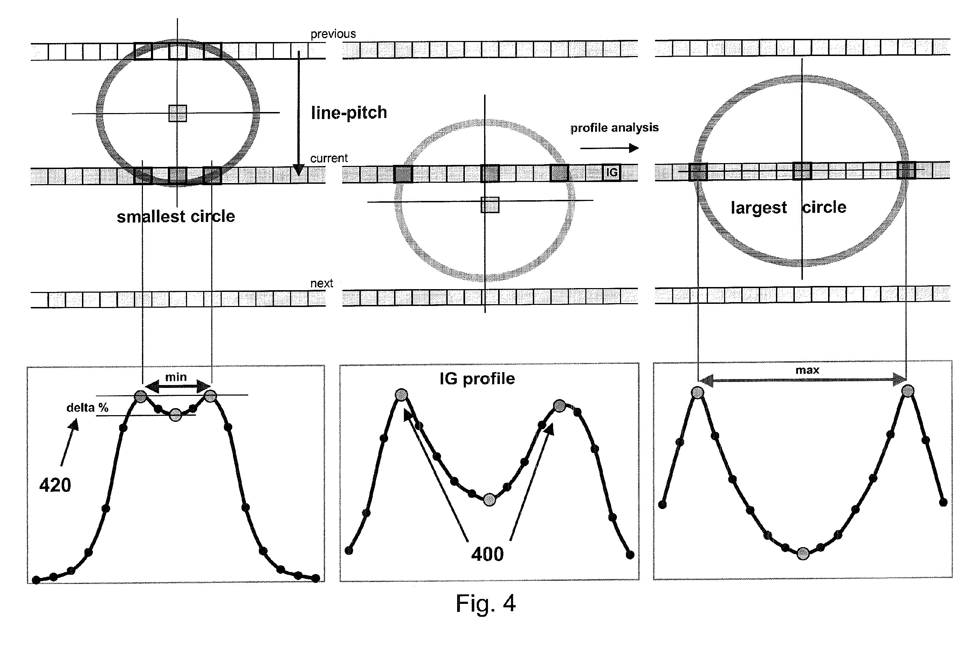

A candidate hole is identified, when the local isotropic gradient line-profile exhibits a typical shape if analysed along the image line as shown in Fig. 4. The scanning software indicates a hole candidate when the curve of the isotropic gradient shows two maxima **400** less than a hole diameter separated from each other, and a minimum between the two maxima that is at least a certain percentage **420** lower than the maxima. This percentage, again, is chosen such that it is impossible to miss a hole.

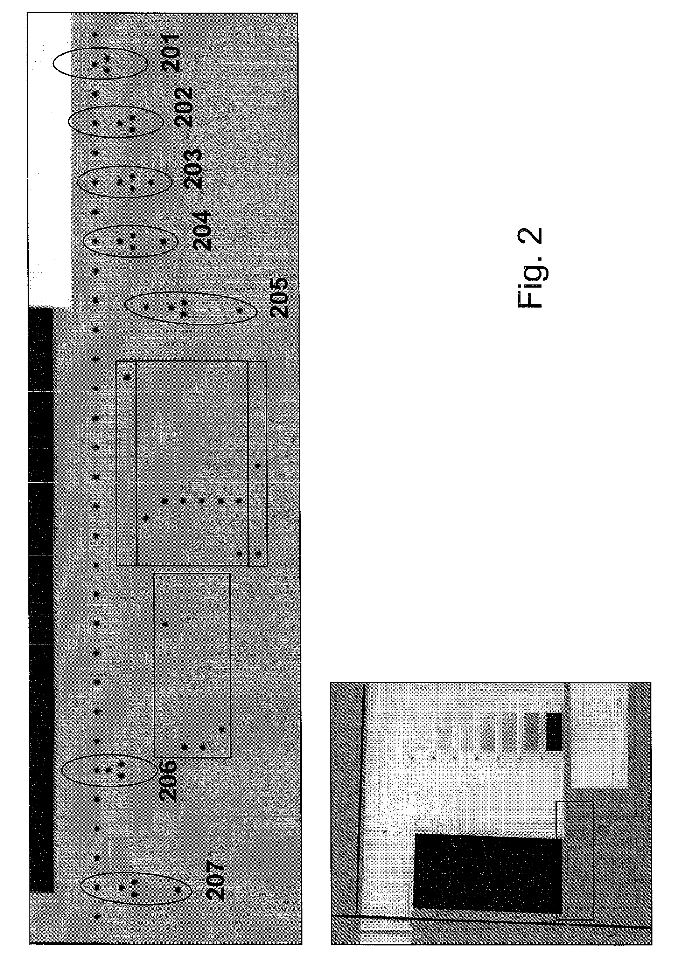

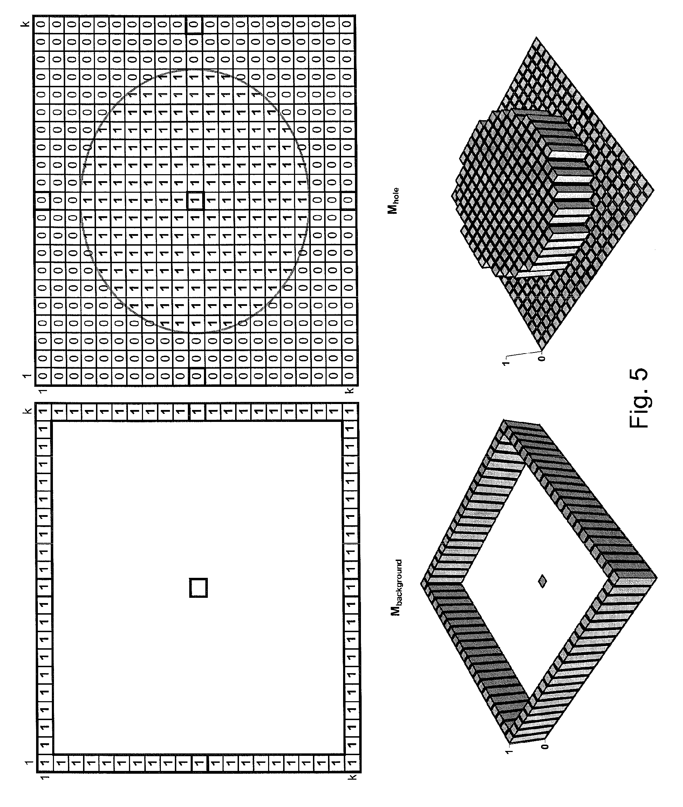

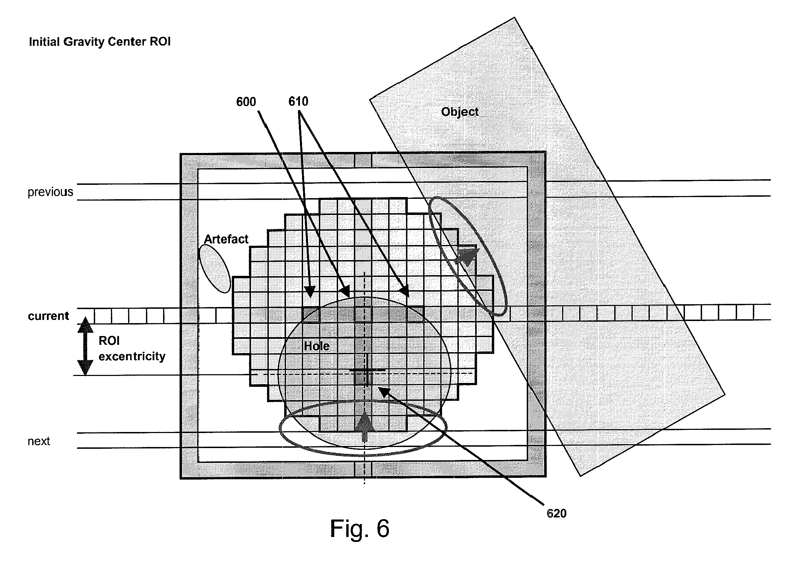

The next step in hole identification is geometrical gravity centre determination, as shown in the flow chart of Fig. 2. Thereto the pixel position at the minimum mentioned above is taken as a first approximation of the geometrical gravity centre of the hole, as shown in Fig. 6. This initial position **600,** as the centre of two edge points **610** of the hole, is always located inside the hole. To find the next approximation of the geometrical gravity centre a background mask ($M_{background}$) is defined around the current approximation as the edge of a square with side $k$, where $k$ is odd and at least twice the diameter of the hole; see Fig. 5. Since the centre of the square is located within the hole, the hole itself completely resides within the background mask. The background mask only consists of the edge pixels of the square. Said background mask cannot intersect the hole itself or another hole, since the distance between two holes is determined to be at least one hole diameter. Said background mask is used to calculate the median value for the square's border-pixels. The median is chosen above the average to optimally suppress the influence of objects or artifacts that might intersect with the background mask, as shown in Fig. 6. Said median back-ground is consecutively subtracted from the dose-linear image data within the local square area, resulting in the superposed signal.

```
k = 2 × (integer fraction of (diameter / pixelsize)) + 1
```

```
Dᵢⱼ = dose-linear Local Area Image Data (k × k matrix)
```

Also a hole mask ($M_{hole}$) is defined as a circular mask around the current approximation of the geometrical gravity centre with a diameter 1,5 times the diameter of the hole. The hole mask contains all the pixels within this circle. Both hole mask and background mask are clarified in Fig. 5. Note, as can be seen in Fig. 6, that there is no guarantee that the hole is completely contained within the area of the hole mask. The sub-pixel geometrical gravity centre **620** over the hole mask is now calculated by means of a standard 2D geometrical gravity point algorithm.

```
GC (cc, rr)ᵢⱼ = Sub-Pixel Geometrical Gravity Centre of ROIᵢⱼ.
```

$$\texttt{cc = i} + \sum_{o=1}^{k}\left(o\cdot\sum_{p=1}^{k}ROI_{ij}(o,p)\right)\Bigg/\sum_{o=1}^{k}\left(\sum_{p=1}^{k}ROI_{ij}(o,p)\right)-(k+1)/2\ .$$

$$\texttt{rr = j} + \sum_{p=1}^{k}\left(p\cdot\sum_{o=1}^{k}ROI_{ij}(o,p)\right)\Bigg/\sum_{o=1}^{k}\left(\sum_{p=1}^{k}ROI_{ij}(o,p)\right)-(k+1)/2\ .$$

pixGC (c,r)$_{ij}$ = pixel containing GC (cc, rr)$_{ij}$.
c = integer fraction of (cc).
r = integer fraction of (rr).

If the hole is contained completely within the hole mask, and if there are no disturbing factors as objects or artifacts intersecting with the hole mask, then this newly calculated geometrical gravity centre is the final one. Otherwise more iterations are necessary, producing a converging sequence of geometrical gravity centres by successive approximation. The iteration process stops when the newly calculated geometrical gravity centre lays within the initial, starting pixel or within the pixel that contains the previously determined geometrical gravity centre.

The sub-pixel geometrical gravity centres of the holes detected can be used to accurately locate the spatial landmarks in the phantom image.

When the centres of all holes are identified, the software looks for clusters of three holes in a predefined composition. This composition is called a centroid and consists of an isosceles triangle, the centres of the holes being the corner points.

A centroid defines the local coordinate system directions and senses as follows: the x-axis is collinear with the smaller base of the triangle, and the y-axis is collinear with the line between the top of the triangle and the middle of its base, which means x-axis and y-axis are perpendicular to each other. The positive direction of the y-axis is the direction from the base to the top of the triangle. The positive sense of the x-axis is a quarter-turn rotated clockwise. These local coordinate systems serve as reference points for local information decoding and are the basis for ROI definition.

Each centroid is characterized by a head and a tail. These are collections of holes on the local y-axis at predetermined distances from the centroid. The position of the centre of every hole in head or tail is part of the code. When the software has detected a centroid, it will look for the accompanying head and tail code-parts. Note that head and/or tail of a centroid could be empty. Since the possible locations of holes in head or tail are at a standard distance from each other, head and tail can be read as a binary number, where the presence of a hole represents a binary '1', and the absence of a hole a binary '0'.

The decoded information in the head is used as a functional descriptor for the data encoded in the tail. A predefined flexible data structure governs the functional link between the head and the tail information. In the preferred embodiment this data structure is a look-up table.

As an example, consider the encoding as depicted in Fig. 2. A centroid without a head **201** is used to code the phantom type. The tail is interpreted as the binary representation of the type number ("0" in **201,** as there are no holes in the tail). A centroid with a head as in **202** points to a sub-target. The tail then specifies the kind of sub-target, i.e.:

- 0 → square
- 1 → rulers
- 2 → step-wedge
- 4 → slits
- 8 → edges

A centroid with a head as in **206** indicates the serial number of the phantom target, the serial number itself being coded in the rectangular frame adjacent to **206.**

It will be recognized that any code scheme would do, and that the particular encoding used in this example is irrelevant to the invention.∎

## Claims

1. A method for embedding information in a phantom target for use with digital radiography systems, the method **characterized in that:**

    (a) the information contained in said phantom target is coded;
    (b) said code is represented by detectable variations in said phantom target; and
    (c) a flexible data structure governs the functional meaning associated with the presence, absence and physical location of said variations.

2. The method of claim 1, wherein the phantom target is used in quality control of digital radiography systems.

3. The method of claims 1 to 2, wherein said detectable variations are alterations in the thickness of the absorbing layer of said phantom target.

4. The method of claim 3, wherein said alterations are holes in the absorbing layer of said phantom target.

5. The method of claim 4, wherein said holes all are circular.

6. The method of claim 5, wherein said holes all have the same diameter.

7. The method of claims 4 to 6, wherein said holes are drilled into the absorbing layer of the phantom target by means of Computer Numerical Control (CNC) techniques.

8. A method for extracting physically embedded, encoded information from a phantom target for use with digital radiography systems, the method **characterized in that:**

    (a) variations in said phantom target are detected by analyzing the digital image of said phantom target;
    (b) the geometrical gravity centre of said detected variations is localized by means of sub-pixel geometrical gravity centre determination; and

(c) said geometrical gravity centres are checked with a flexible data structure governing the functional meaning associated with the presence, absence and physical location of said variations;

9. The method of claim 8, wherein the detection of said variations in step (a) is **characterized in that:**

(i) the digital image of said phantom target is first dose-linearized and offset subtracted;
(ii) a gradient image of the digital image is calculated;
(iii) edge analysis of the gradient image is performed to establish the starting points for the geometrical gravity centre determination of said variations;
(iv) when a marker candidate is found, the geometrical gravity centre of the variation is determined;
(v) the variation said geometrical gravity centre belongs to then is checked against a set of given conditions that are prerequisites for a variation to be accepted as a marker variation;
(vi) if accepted, the marker variation geometrical gravity centre is added to a marker list if said marker is new, i.e. if it is not contained in said marker list already;

10. The method of claim 9, wherein the gradient image of (ii) is an isotropic gradient image.

11. The method of claims 9 to 10, where the set of given conditions of (v) determines that a variation must be a circular hole with a given diameter to be accepted as a marker.

12. A system for embedding information in a phantom target for use with digital radiography systems, the system **characterized by**:

(a) a phantom target containing detectable variations; and
(b) a flexible data structure governing the functional meaning associated with the presence, absence and physical location of said variations.

13. The system of claim 12, wherein the phantom target is used in quality control of digital radiography systems.

14. The system of claims 12 to 13, wherein said detectable variations are alterations in the thickness of the absorbing layer of said phantom target.

15. The system of claim 14, wherein said alterations are holes in the absorbing layer of said phantom target.

16. The system of claim 15, wherein said holes all are circular.

17. The system of claim 16, wherein said holes all have the same diameter.

18. The system of claims 15 to 17, wherein said holes are drilled into the absorbing layer of the phantom target by means of Computer Numerical Control (CNC) techniques.

19. A system for extracting physically embedded, encoded information from a phantom target for use with digital radiography systems, the system **characterized by** a processor for:

(a) detecting variations in said phantom target by analyzing the digital image of said phantom target;
(b) localizing the geometrical gravity centre of said detected variations by means of sub-pixel geometrical gravity centre determination; and
(c) checking said geometrical gravity centres with a flexible data structure governing the functional meaning associated with the presence, absence and physical location of said alterations;

20. The system of claim 19, wherein the detection in (a) is **characterized by**:

(i) the digital image of said phantom target is first dose-linearized and offset subtracted;
(ii) a gradient image of the digital image is calculated;
(iii) edge analysis of the gradient image is performed to establish the starting points for the geometrical gravity centre determination of said variations;
(iv) when a marker candidate is found, the geometrical gravity centre of the variation is determined;

(v) the variation said geometrical gravity centre belongs to is checked against a set of given conditions that are prerequisites for a variation to be accepted as a marker variation;

(vi) if accepted, the marker variation geometrical gravity centre is added to a marker list if said marker is new, i.e. if it is not contained in said marker list already;

21. The system of claim 20, wherein the gradient image of (ii) is an isotropic gradient image.

22. The system of claims 20 to 21, where the set of given conditions of (v) determines that a variation must be a circular hole with a given diameter to be accepted as a marker.

23. A computer program product adapted to carry out the steps of any of claims 1 to 11 when run on a computer.

24. A computer readable carrier medium comprising computer executable program code adapted to carry out the steps of any of claims 1 to 11.

# Hole detection and sub-pixel gravity center calculation

start

raw image →

calculate the dose-linear signal for each image pixel

dose-linear image

calculate the Isotropic Gradient for each image pixel

Isotropic Gradient image

locate the first line and calculate the line-pitch
for Isotropic Gradient profile analysis

for each analysis-line

- Analyze the isotropic gradient line-profile
to detect candidate hole signal-clusters
- Determine the starting-points
for local gravity center determination

for each candidate hole

- Calculate the sub-pixel gravity center GC (cc,rr)
- Check if the candidate hole is circular and has the right radius.
- Add GC(cc, rr) to the image's holes list if it is a new hole.

next candidate

next line

stop →

Holes List
GC(cc,rr)

## Fig. 1

Fig. 2

EP 2 028 621 A1

$A_{ij}$

| | i-1 | i | i+1 |
|---|---|---|---|
| j-1 | d | c | b |
| j | e | $p_{ij}$ | a |
| j+1 | f | g | h |

K

| | i-1 | i | i+1 |
|---|---|---|---|
| j-1 | $-2^{-1.5}$ | $2^{-1}$ | $2^{-1.5}$ |
| j | $-2^{-1}$ | 0 | $2^{-1}$ |
| j+1 | $-2^{-1.5}$ | $-2^{-1}$ | $2^{-1.5}$ |

$M_0$

| | i-1 | i | i+1 |
|---|---|---|---|
| j-1 | 0 | 0 | 0 |
| j | 1 | 0 | 1 |
| j+1 | 0 | 0 | 0 |

$M_{45}$

| | i-1 | i | i+1 |
|---|---|---|---|
| j-1 | 0 | 0 | 1 |
| j | 0 | 0 | 0 |
| j+1 | 1 | 0 | 0 |

$M_{90}$

| | i-1 | i | i+1 |
|---|---|---|---|
| j-1 | 0 | 1 | 0 |
| j | 0 | 0 | 0 |
| j+1 | 0 | 1 | 0 |

$M_{135}$

| | i-1 | i | i+1 |
|---|---|---|---|
| j-1 | 1 | 0 | 0 |
| j | 0 | 0 | 0 |
| j+1 | 0 | 0 | 1 |

300

310

320

K

$M_0$

$M_{45}$

$M_{90}$

$M_{135}$

Fig. 3

Fig. 4

$M_{background}$

$M_{hole}$

Fig. 5

Fig. 6

Fig. 7

dose-linear signal
$D_{cr}$

object

superposed signal
$D_{cr} - BG_{cr}$

object

circulary masked signal
$ROI_{cr}$

artefact

artefact

hole

Fig. 8

EP 2 028 621 A1

Projection Radiography CR / DR Imaging Chain

Fig. 9

**European Patent Office**

**EUROPEAN SEARCH REPORT**

Application Number

EP 07 11 4581

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 5 822 396 A (NAVAB NASSIR [US] ET AL) 13 October 1998 (1998-10-13)<br><br>* abstract *<br><br>* column 1, line 37 - column 2, line 5 *<br>* column 2, line 62 - column 3, line 3 *<br>* column 5, line 12 - column 6, line 45 *<br>* column 8, line 57 - line 60 *<br>* figures 2-6 * | 1-8, 12-19, 23,24<br>9-11, 20-22 | INV.<br>G06T7/60<br>A61B6/00 |
| X<br><br>Y | US 2003/058999 A1 (MITSCHKE MATTHIAS [DE] ET AL) 27 March 2003 (2003-03-27)<br><br>* abstract *<br><br>* paragraph [0013] - paragraph [0014] *<br>* paragraph [0028] - paragraph [0032] *<br>* paragraph [0037] *<br>* paragraph [0040] - paragraph [0047] *<br>* figure 3 * | 1-3,8, 12-14, 19,23,24<br>9-11, 20-22 | |
| X | EP 1 074 220 A (GEN ELECTRIC [US]) 7 February 2001 (2001-02-07)<br><br>* abstract *<br>* figure 3 * | 1-4,7, 12-15, 18,23,24 | TECHNICAL FIELDS SEARCHED (IPC)<br><br>G06T<br>A61B |
| Y | US 6 859 555 B1 (FANG TONG [US] ET AL) 22 February 2005 (2005-02-22)<br>* abstract *<br>* column 4, line 60 - line 67 *<br>* column 5, line 31 - column 7, line 35 *<br>* figures 1,2 * | 9-11, 20-22 | |

-/--

The present search report has been drawn up for all claims

| Place of search | Date of completion of the search | Examiner |
|---|---|---|
| Munich | 14 January 2008 | Engels, Angela |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**European Patent Office**

**EUROPEAN SEARCH REPORT**

Application Number

EP 07 11 4581

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GB 2 276 446 A (HONDA MOTOR CO LTD [JP]) 28 September 1994 (1994-09-28) * abstract * * page 1, line 1 - page 2, line 8 * * page 2, line 27 - page 3, line 3 * * page 4, line 26 - page 5, line 1 * ----- | 9,20 | |
| | | | **TECHNICAL FIELDS SEARCHED (IPC)** |

The present search report has been drawn up for all claims

| Place of search | Date of completion of the search | Examiner |
|---|---|---|
| Munich | 14 January 2008 | Engels, Angela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                   

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 11 4581

This annex lists the patent family members relating to the patent documents cited in  the above-mentioned European search report.
The members are as contained in the European Patent Office EDP file on
The European Patent Office is in no way liable for these particulars which are merely given for the purpose of information.

14-01-2008

| Patent document cited in search report | | Publication date | Patent family member(s) | | Publication date |
|---|---|---|---|---|---|
| US 5822396 | A | 13-10-1998 | JP | 3881696 B2 | 14-02-2007 |
| | | | WO | 9723164 A1 | 03-07-1997 |
| US 2003058999 | A1 | 27-03-2003 | DE | 10140867 A1 | 03-04-2003 |
| EP 1074220 | A | 07-02-2001 | DE | 60015449 D1 | 09-12-2004 |
| | | | DE | 60015449 T2 | 08-12-2005 |
| | | | JP | 2001104283 A | 17-04-2001 |
| | | | US | 2002061502 A1 | 23-05-2002 |
| US 6859555 | B1 | 22-02-2005 | NONE | | |
| GB 2276446 | A | 28-09-1994 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1369084 A
- EP 1380259 A
- US 6369402 B

**Non-patent literature cited in the description**

- **R. SCHAETZING ; R. FASBENDER ; P. KERSTEN.** New high-speed scanning technique for Computed Radiography. *Proc. SPIE,* vol. 4682, 511-520